# EUROPÄISCHE PATENTSCHRIFT

(11) **EP 2 033 592 B1**
(45) Veröffentlichungstag und Bekanntmachung des Hinweises auf die Patenterteilung: **24.04.2019**
(21) Anmeldenummer: 08104814.2
(22) Anmeldetag: 22.07.2008
(51) Int. Cl.: G08C 17/00, A61B 34/00, A61B 90/00, A61B 6/00, G06F 19/00

(54) **Steuerungsvorrichtung und Verfahren zur Steuerung einer medizinischen Diagnostik- und/oder Therapieanlage sowie medizinische Diagnostik- und/oder Therapieanlage**
Control device and method for controlling a medical diagnosis and/or therapy assembly and medical diagnosis and/or therapy assembly
Dispositif de commande et procédé de commande d'une installation médicale de diagnostic et/ou de thérapie et installation médicale de diagnostic et/ou de thérapie

(30) Priorität: 06.09.2007 DE 102007042337
(43) Veröffentlichungstag der Anmeldung: 11.03.2009
(73) Patentinhaber: Varian Medical Systems Particle Therapy GmbH, 53842 Troisdorf (DE)
(72) Erfinder: Rietzel, Eike, Dr., 64289 Darmstadt (DE); Sommer, Andres, 91094 Langensendelbach (DE)
(74) Vertreter: Kirchner, Veit

(56) Entgegenhaltungen:
- WO-A-2005/018242
- WO-A-2007/009881
- DE-A1- 10 116 870
- DE-A1-102004 048 067
- US-A1- 2002 087 048
- US-A1- 2005 065 417
- Anonymous: "Clinac Downtime Summary KFJ", , 14 June 2006 (2006-06-14), XP055213192, Retrieved from the Internet: URL:http://www.wienkav.at/kav/kfj/91033454 /physik/html/downtime.htm [retrieved on 2015-09-14]
- Anonymous: "Downtime Details Clinac Green (S/N 156)", , 18 September 1999 (1999-09-18), XP055213183, Retrieved from the Internet: URL:http://www.wienkav.at/kav/kfj/91033454 /physik/html/downtime_green.htm [retrieved on 2015-09-14]
- Jacob Abderhalden ET AL: "The IRO Medical Physics and Engineering Group (MPEG) Members of the Group", , 14 June 2006 (2006-06-14), XP055213190, Retrieved from the Internet: URL:http://web.archive.org/web/20060114205 357/http://www.wienkav.at/kav/kfj/91033454 /physik/irohome.htm [retrieved on 2015-09-14]
- Merryl Ginsberg: "CENTERLINE MAGAZINE", , 31 October 2004 (2004-10-31), XP055471405, Retrieved from the Internet: URL:https://www.varian.com/oncology/center line/2004-10 [retrieved on 2018-04-30]

## Beschreibung

Steuerungsvorrichtung und Verfahren zur Steuerung einer medizinischen Diagnostik- und/oder Therapieanlage sowie medizinische Diagnostik- und/oder Therapieanlage
Die Erfindung betrifft eine Steuerungsvorrichtung für eine medizinische Diagnostik- und/oder Therapieanlage sowie ein Verfahren zur Steuerung einer solchen. Weiterhin betrifft die Erfindung eine medizinische Diagnostik- und/oder Therapieanlage mit einer derartigen Steuerungsvorrichtung. Der Gegenstand der Erfindung findet insbesondere im Rahmen der Partikeltherapie Einsatz, bei der eine Vielzahl von steuerbaren Elementen koordiniert miteinander gesteuert werden müssen.

Ein Funk-Bediensystem und ein Betriebsverfahren desselben für ein medizinisches Gerät sind aus WO 2005/018242 bekannt. Das Funk-Bediensystem kann eine Steuerungskomponente für das medizinisches Gerät sein und bestimmte Steuervorgänge können durch das Funk-Bediensystem ausgeführt werden wenn sich ein Bedienteil des Funk-Bediensystems in einer gewissen Reichweite von einem Funk-Basisstation befindet.

Die folgende Beschreibung wird am Beispiel der Partikeltherapie bzw. anhand einer Partikeltherapieanlage vorgenommen, kann jedoch ohne weiteres auch auf andere medizinische Diagnostik- und/oder Therapieanlagen übertragen werden.

Die Partikeltherapie ist ein etabliertes Verfahren zur Behandlung von Gewebe, insbesondere von Tumorerkrankungen. Bestrahlungsverfahren, wie sie in der Partikeltherapie eingesetzt werden, finden jedoch auch in nicht-therapeutischen Gebieten Anwendung. Hierzu gehören beispielsweise Forschungsarbeiten im Rahmen der Partikeltherapie, die an nicht-lebenden Phantomen oder Körpern durchgeführt werden, Bestrahlungen von Materialien, etc. Hierbei werden üblicherweise geladene Partikel auf hohe Energien beschleunigt, zu einem Partikelstrahl geformt und über ein Hochenergiestrahltransportsystem zu einem oder mehreren Bestrahlungsräumen geführt. In einem dieser Bestrahlungsräume wird das zu bestrahlende Objekt mit dem Partikelstrahl bestrahlt.

Bei einer Partikeltherapieanlage müssen verschiedene Komponenten genau aufeinander abgestimmte, verschiedene Arbeitsschritte ausführen. Die unterschiedlichen Komponenten werden dabei üblicherweise von einer Steuerungsvorrichtung gesteuert, durch die ein korrektes Zusammenspiel der einzelnen Komponenten und damit ein korrektes Funktionieren der Partikeltherapieanlage gewährleistet ist.

Ein Teil der Steuervorgänge einer Partikeltherapieanlage kann dabei durch einen Anwender mithilfe eines mobilen Handbedienteils durchgeführt werden. Insbesondere, wenn ein Anwender sich in einem Behandlungsraum einer Partikeltherapieanlage befindet - beispielsweise um einen Patienten für eine folgende Bestrahlungssitzung vorzubereiten - kann ein derartiges Handbedienteil vorteilhaft eingesetzt werden. Ein Anwender kann beispielsweise über das Handbedienteil die Patientenpositioniervorrichtung bedienen, auf der sich der Patient befindet, oder Einstellungen an der Partikeltherapieanlage über das Handbedienteil vornehmen. Das mobile Handbedienteil hat dabei den Vorteil, dass der Anwender die Steuerung von verschiedenen Orten aus durchführen kann, beispielsweise in unmittelbarer Nähe des Patienten, ohne jeweils zu einem fest installierten Steuergerät hingehen zu müssen.

Es ist die Aufgabe der Erfindung, eine Steuerungsvorrichtung zur Steuerung einer medizinischen Diagnostik- und/oder Therapieanlage anzugeben, mit der ein sicheres Betreiben der medizinischen Diagnostik- und/oder Therapieanlage auch bei Verwendung eines mobilen Handbedienteils ermöglicht wird. Weiterhin ist es die Aufgabe der Erfindung, eine medizinische Diagnostik- und/oder Therapieanlage mit einer derartigen Steuerungsvorrichtung anzugeben. Weiterhin ist es die Aufgabe der Erfindung, ein Verfahren zur Steuerung einer medizinischen Diagnostik- und/oder Therapieanlage anzugeben, das eine sichere Steuerung der medizinischen Diagnostik- und/oder Therapieanlage auch bei Verwendung eines mobilen Handbedienteils ermöglicht.

Die Aufgabe der Erfindung wird gelöst durch eine Steuerungsvorrichtung für eine Partikeltherapieanlage nach Anspruch 1, durch eine Partikeltherapieanlage nach Anspruch 6 sowie durch ein Verfahren zur Steuerung einer Partikeltherapieanlage nach Anspruch 7. Vorteilhafte Weiterbildungen finden sich in den Merkmalen der abhängigen Ansprüche.

Die erfindungsgemäße Steuerungsvorrichtung für eine medizinische Diagnostik- und/oder Therapieanlage umfasst
- eine Steuerungseinheit, mit welcher Steuervorgänge zur Steuerung der medizinischen Diagnostik- und/oder Therapieanlage durchführbar sind,
- ein mobiles Handbedienteil, mit dem zumindest ein steuerbares Element der medizinischen Diagnostik- und/oder Therapieanlage durch einen Anwender steuerbar ist,
wobei die Steuerungsvorrichtung derart ausgebildet ist, dass ein Teil der Steuervorgänge erst dann durch die Steuerungseinheit durchführbar ist, wenn sich das mobile Handbedienteil an einem vordefinierten Ort befindet.

Bestimmte Steuerungsvorgänge sind folglich gesperrt und können durch die Steuerungseinheit nicht ausgeführt werden, solange sich das mobile Handbedienteil nicht an dem vordefinierten Ort befindet. Auf diese Weise wird die Sicherheit beim Betreiben der medizinischen Diagnostik- und/oder Therapieanlage erhöht gegenüber einer Anlage, bei der ein derartiger Kontrollmechanismus nicht zur Verfügung steht.

In letzterem Falle kann es passieren, dass ein Anwender, der zuvor eine Steuerung mit dem mobilen Handbedienteil vorgenommen hat, das mobile Handbedienteil an einer ungünstigen Stelle positioniert. Durch Steuervorgänge, die anschließend folgen, kann es passieren, dass das mobile Handbedienteil unbeabsichtigt beschädigt oder gar derart manipuliert wird, dass es in unbeabsichtigter Weise Steuerkommandos erzeugt. Wenn beispielsweise das mobile Handbedienteil auf einer Patientenliege vergessen wird und der Anwender anschließend den Raum verlässt, damit eine Bildgebungsuntersuchung oder eine Bestrahlungssitzung starten kann, ist es möglich, dass das Handbedienteil durch Röntgenstrahlen oder durch den Behandlungsstrahl beschädigt wird oder dass Fehlfunktionen ausgelöst werden. Dies kann zu einer erheblichen Beeinträchtigung der Sicherheit führen.

Durch die erfindungsgemäße Steuerungsvorrichtung ist gewährleistet, dass sich das mobile Handbedienteil an einem vordefinierten Ort befindet, bevor bestimmte Steuerungsvorgänge durch die Steuerungseinheit durchgeführt werden können. Auf diese Weise kann sichergestellt werden, dass das mobile Handbedienteil nicht durch eine Steuerung der medizinischen Diagnostik- und/oder Therapieanlage beschädigt wird. Die Sicherheit beim Betreiben der medizinischen Diagnostik- und/oder Therapieanlage und insbesondere die Patientensicherheit werden dadurch erheblich erhöht.

Der vordefinierte Ort innerhalb der medizinischen Diagnostik- und/oder Therapieanlage kann sich z.B. innerhalb eines Diagnose- und/oder Therapieraums befinden, insbesondere außerhalb der Reichweite von beweglichen Elementen innerhalb des Diagnose- und/oder Therapieraums und/oder insbesondere außerhalb des Wirkbereichs von Diagnose- und/oder Therapievorrichtungen. Der vordefinierte Ort kann sich aber auch beispielsweise außerhalb des Diagnose- und/oder Therapieraums befinden. Die Steuerungseinheit befindet sich üblicherweise außerhalb des Diagnose- und/oder Therapieraums.

Die Steuerungsvorrichtung - und insbesondere die Steuerungseinheit - muss dabei nicht eine einzige in sich abgeschlossene Einheit sein. Sie kann auch mehrere verteilte Untereinheiten umfassen, von denen jeweils eine Steuerung unterschiedlicher Elemente vorgenommen wird.

In einer vorteilhaften Ausführungsvariante umfasst die Steuerungsvorrichtung ein Positionsüberwachungssystem, mit welchem ermittelt werden kann, ob sich das mobile Handbedienteil an dem vordefinierten Ort befindet oder nicht. Als Positionsüberwachungssystem bzw. Ortungssystem können dabei bekannte Positionsüberwachungssysteme bzw. Ortungssysteme eingesetzt werden, beispielsweise mit elektromagnetischen Wellen oder akustischen Wellen arbeitende Positionsüberwachungssysteme.

Die Positionsüberwachung kann dabei kontinuierlich erfolgen oder lediglich punktuell, d.h. unmittelbar bei der Durchführung des Steuervorganges, der nur dann durchführbar ist, wenn das mobile Handbedienteil sich an dem vordefinierten Ort befindet.

In einer vorteilhaften Ausführungsvariante kann die Steuerungsvorrichtung eine Halterungsvorrichtung für das mobile Handbedienteil aufweisen. Der vordefinierte Ort entspricht dann dem Platz in der Halterungsvorrichtung. Das bedeutet, dass der Teil der Steuervorgänge erst dann durchführbar ist, wenn sich das mobile Handbedienteil in der Halterungsvorrichtung befindet. Auf diese Weise kann auf einfache Weise sichergestellt werden, dass sich das mobile Handbedienteil an seinem korrekten Platz befindet.

Das mobile Handbedienteil ist insbesondere als schnurloses Handbedienteil ausgebildet, so dass Steuerkommandos des Handbedienteils drahtlos an eine Steuerungseinheit übertragen werden.

In einer vorteilhaften Ausführungsvariante kann der Teil der Steuervorgänge, der durch die Steuerungseinheit erst dann ausführbar ist, wenn sich das mobile Handbedienteil an dem vordefinierten Ort befindet, eine Steuerung des steuerbaren Elements umfassen, das durch einen Anwender mit dem mobilen Handbedienteil gesteuert werden kann. Auf diese Weise kann eine Hierarchie der Steuerung des steuerbaren Elements auf einfache Weise geregelt werden. Solange sich das mobile Handbedienteil nicht an dem vordefinierten Ort befindet, da beispielsweise ein Anwender das mobile Handbedienteil bedient, ist eine Steuerung des steuerbaren Elements durch die Steuerungseinheit gesperrt. Das steuerbare Element kann dann im regulären Betrieb nur durch das mobile Handbedienteil bedient werden. Erst wenn sich das mobile Handbedienteil an dem vordefinierten Ort - beispielsweise in einer Halterungsvorrichtung - befindet, kann die Steuerung des steuerbaren Elements durch die Steuerungseinheit übernommen werden. Interferierende oder gegensätzliche Steuerungsanweisungen können auf diese Weise vermieden werden. Das steuerbare Element kann insbesondere ein bewegbares Element in einem Diagnose- und/oder Therapieraum sein, insbesondere eine Patientenpositioniervorrichtung und/oder eine Bildgebungsvorrichtung. In einer Partikeltherapieanlage kann das bewegbare Element eine insbesondere um einen Bestrahlungsraum rotierbare Gantry sein.

Insbesondere, wenn durch das mobile Handbedienteil Komponenten wie z.B. die Bildgebungsvorrichtung oder die Patientenpositioniervorrichtung bewegt werden, ist hierdurch auch ein Anwender, der das mobile Handbedienteil bedient, vor einer unbeabsichtigten Steuerung dieser Komponenten von der Steuereinheit geschützt. Eine Steuerung der Komponenten durch die Steuereinheit könnte andernfalls einen Anwender gefährden, wenn er sich im Bewegungsradius der beweglichen Komponenten befindet.

In einer anderen vorteilhaften Ausführungsvariante kann der Teil der Steuervorgänge, der durch die Steuerungseinheit erst dann ausführbar ist, wenn sich das mobile Handbedienteil an dem vordefinierten Ort befindet, eine Steuerung von einem weiteren steuerbaren Element umfassen, das nicht durch einen Anwender mit dem mobilen Handbedienteil gesteuert werden kann. Auf diese Weise kann eine Hierarchie der Steuerung von verschiedenen Elementen, die von verschiedenen Einheiten - dem mobilen Handbedienteil und der Steuerungseinheit - gesteuert werden, auf einfache Weise geregelt werden, so dass sich die Sicherheit bei der Steuerung der medizinischen Diagnostik- und/oder Therapieanlage erhöht. Steuerbare Elemente, die üblicherweise nicht von einem mobilen Handbedienteil über Steuervorgänge gesteuert werden können, sind bei einer Partikeltherapie insbesondere Beschleunigereinheiten zu Beschleunigung von Partikeln, ein Hochenergiestrahl-Transportsystem, etc.

In einer vorteilhaften Ausführungsvariante ist die Steuerungsvorrichtung als Steuerungsvorrichtung für eine Partikeltherapieanlage ausgebildet, wobei einer der Steuervorgänge der Steuerungseinheit die Abgabe eines Strahlfreigabesignals ist. Ein Strahlfreigabesignal liegt in einer Partikeltherapieanlage üblicherweise dann vor, wenn alle relevanten Sicherheitsschecks vor einer geplanten Bestrahlung durchgeführt worden sind. Erst bei Vorliegen eines Strahlfreigabesignals kann tatsächlich eine Bestrahlung eines zu bestrahlenden Objekts stattfinden. Bei dieser Ausführungsvariante kann ein Strahlfreigabesignal erst dann durch die Steuerungseinheit abgegeben werden, wenn sich das mobile Handbedienteil an dem vordefinierten Ort befindet. Auf diese Weise ist sichergestellt, dass das mobile Handbedienteil nicht fälschlicherweise im Bereich des Behandlungsstrahls vergessen wird, so dass es bei einer anschließenden Bestrahlung beschädigt würde.

Die erfindungsgemäße medizinische Diagnostik- und/oder Therapieanlage umfasst eine Diagnostik- bzw. Therapievorrichtung, die von einer Steuerungsvorrichtung nach einem der Ansprüche 1 bis 7 gesteuert wird, sowie das steuerbare Element, das durch das mobile Handbedienteil der Steuerungsvorrichtung gesteuert wird.

Bei dem erfindungsgemäßen Verfahren zur Steuerung einer medizinischen Diagnostik- und/oder Therapieanlage, werden mehrere Steuervorgänge zur Steuerung der medizinischen Diagnostik- und/oder Therapieanlage durchgeführt. Das Verfahren zur Steuerung ist dabei derart ausgebildet, dass ein Teil der Steuervorgänge erst dann durchführbar ist, wenn sich ein mobiles Handbedienteil, mit dem zumindest ein steuerbares Element durch einen Anwender steuerbar ist, an einem vordefinierten Ort befindet.

Ausführungsformen der Erfindung und vorteilhafte Weiterbildungen gemäß den Merkmalen der abhängigen Ansprüche werden anhand der folgenden Zeichnung näher erläutert, ohne jedoch darauf beschränkt zu sein. Es zeigen:
Fig. 1 einen schematischen Überblick über eine Partikeltherapieanlage,
Fig. 2 einen schematischen Überblick über einen Bestrahlungsraum, in welchem ein Anwender bestimmte Steuervorgänge mit einem mobilen Handbedienteil ausführen kann, und
Fig. 3 einen schematischen Überblick über Steuervorgänge, die bei der Durchführung einer Ausführungsform des erfindungsgemäßen Verfahrens durchgeführt werden.

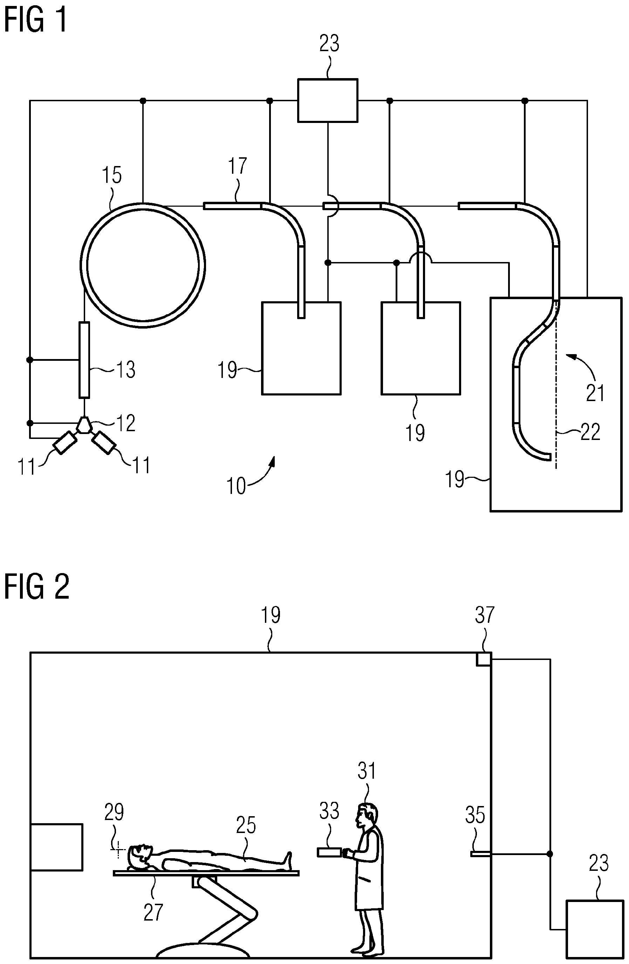

Fig. 1 zeigt einen schematischen Überblick über den Aufbau einer Partikeltherapieanlage 10. In einer Partikeltherapieanlage 10 erfolgt insbesondere eine Bestrahlung eines Körpers, insbesondere eines tumorerkrankten Gewebes, mit einem Partikelstrahl.

Als Partikel werden vornehmlich Ionen wie beispielsweise Protonen, Pionen, Heliumionen, Kohlenstoffionen oder andere Ionensorten eingesetzt. Üblicherweise werden derartige Partikel in einer Partikelquelle 11 erzeugt. Wenn, wie in Figur 1 dargestellt, zwei Partikelquellen 11 vorhanden sind, die zwei verschiedene Ionensorten erzeugen, kann zwischen diesen beiden Ionensorten innerhalb eines kurzen Zeitintervalls umgeschaltet werden. Dazu wird beispielsweise ein Schaltmagnet 12 verwendet, der zwischen den Ionenquellen 11 einerseits und einem Vorbeschleuniger 13 andererseits angeordnet ist. Z.B. kann hierdurch die Partikeltherapieanlage 10 mit Protonen und mit Kohlenstoffionen gleichzeitig betrieben werden.

Die von der oder einer der Ionenquellen 11 erzeugten und gegebenenfalls mit dem Schaltmagneten 12 ausgewählten Ionen werden in dem Vorbeschleuniger 13 auf ein erstes Energieniveau beschleunigt. Der Vorbeschleuniger 13 ist beispielsweise ein Linearbeschleuniger (LINAC für engl.: "LINear ACcelerator"). Anschließend werden die Partikel in einen Beschleuniger 15, beispielsweise ein Synchrotron oder Zyklotron, eingespeist. In dem Beschleuniger 15 werden sie auf hohe Energien, wie sie zur Bestrahlung nötig sind, beschleunigt. Nachdem die Partikel den Beschleuniger 15 verlassen, führt ein Hochenergiestrahl-Transportsystem 17 den Partikelstrahl zu einem oder mehreren Bestrahlungsräumen 19. In einem Bestrahlungsraum 19 werden die beschleunigten Partikel auf einen zu bestrahlenden Körper gerichtet. Je nach Ausgestaltung erfolgt dies von einer festen Richtung (in so genannten "fixed beam"-Räumen) aus oder aber über eine um eine Achse 22 bewegliche rotierbare Gantry 21 von verschiedenen Richtungen aus.

Der anhand der Figur 1 dargestellte Grundaufbau einer Partikeltherapieanlage 10 ist typisch für viele Partikeltherapieanlagen, kann aber auch hiervon abweichen; beispielsweise muss, je nach Beschleunigung der Partikel, eine Bestrahlungsvorrichtung nicht als Partikeltherapieanlage angeordnet sein.

Die Partikeltherapieanlage 10 umfasst dabei eine Vielzahl von steuerbaren Elementen, die sich sowohl außerhalb von Bestrahlungsräumen 19 - wie z.B. die Beschleuniger- und Strahlführungskomponenten - als auch innerhalb von Bestrahlungsräumen 19 - wie z.B. Patientenpositioniervorrichtungen und Bildgebungsvorrichtungen - befinden. Die steuerbaren Elemente werden unter anderem von einer Steuerungseinheit 23 gesteuert. Die Steuerungseinheit 23 muss dabei nicht als einzelne abgeschlossene Einheit aufgebaut sein, sondern kann eine Vielzahl von Untereinheiten umfassen, die jeweils verschiedene steuerbare Elemente steuern.

Fig. 2 zeigt einen schematischen Überblick über einen Bestrahlungsraum 19. In einem Bestrahlungsraum 19 kann ein zu bestrahlendes Objekt, z.B. ein Patient 25, auf einer Positioniervorrichtung 27 und mit der Positioniervorrichtung 27 in Bezug auf ein Isozentrum 29 positioniert werden, so dass eine räumlich definierte Beziehung zwischen einem Partikelstrahl und dem zu bestrahlenden Objekt eingehalten werden kann.

Bestimmte steuerbare Elemente, wie z.B. die Positioniervorrichtung 27, können zusätzlich von einem Anwender 31 mithilfe eines mobilen Handbedienteils 33 drahtlos gesteuert werden. Dies erlaubt einem Anwender 31 beispielsweise, in der Nähe des Patienten 25 zu bleiben, um beispielsweise den Patienten 25 zu versorgen, und gleichzeitig die Positioniervorrichtung 27 zu steuern.

In dem Bestrahlungsraum 19 befindet sich zusätzlich eine Halterungsvorrichtung 35 für das mobile Handbedienteil 33, die gleichzeitig eine Ladeschale für das mobile Handbedienteil 33 ist, so dass ein Akku des mobilen Handbedienteils 33 aufgeladen werden kann. Die Halterungsvorrichtung 35 ist dabei derart ausgebildet, dass sie erkennt, ob sich das mobile Handbedienteil 33 in der Halterungsvorrichtung 35 befindet oder nicht. Diese Information wird der Steuerungseinheit 23 bereitgestellt. Bestimmte Steuervorgänge, die durch die Steuerungseinheit 23 ausgeführt werden, können erst dann durch die Steuerungseinheit 23 ausgeführt werden, wenn sich das mobile Handbedienteil 33 in der Halterungsvorrichtung 35 befindet.

Einer dieser Steuervorgänge kann beispielsweise die Abgabe eines Strahlfreigabesignals von der Steuerungseinheit 23 sein. Ein Strahlfreigabesignal bewirkt, dass ein Partikelstrahl tatsächlich auf ein zu bestrahlendes Objekt gerichtet werden kann. Ohne Vorliegen eines Strahlfreigabesignals ist die Applikation eines Partikelstrahls blockiert. In der hier beschriebenen Ausführungsform der Steuerungsvorrichtung muss sich folglich das mobile Handbedienteil 33 an einem vordefinierten Ort befinden - wie hier gezeigt in der Halterungsvorrichtung 35 -, um ein Strahlfreigabesignal zu ermöglichen. Neben dieser Bedingung müssen üblicherweise weitere Bedingungen erfüllt sein, damit ein Strahlfreigabesignal abgegeben werden kann, wie beispielsweise ein korrektes Funktionieren von Beschleunigungs- und Strahlführungskomponenten. Auf diese Weise wird sichergestellt, dass sich das mobile Handbedienteil 33 an einem sicheren Ort befindet, bevor eine Bestrahlung beginnt und nicht versehentlich in der Nähe des Patienten 25, d.h. in der Nähe des Partikelstrahls vergessen wird.

Ein anderer dieser Steuervorgänge kann beispielsweise die Steuerung der Positioniervorrichtung 27 durch die Steuerungseinheit 23 sein. In diesem Fall kann die Positioniervorrichtung 27 erst dann durch die Steuerungseinheit 23 gesteuert werden, wenn sich das mobile Handbedienteil 33 an dem vordefinierten Ort befindet. Auf diese Weise wird sichergestellt, dass bei einer Steuerung der Positioniervorrichtung 27 keine sich widersprechenden Steuerbefehle gegeben werden, da eine Hierarchie bezüglich der Steuerung der Positioniervorrichtung 27 eindeutig festgelegt ist.

Alternativ und/oder zusätzlich kann die Position des mobilen Handbedienteils 33 beispielsweise durch ein Positionsüberwachungssystem 37, das sich im Bestrahlungsraum befindet, ermittelt werden.

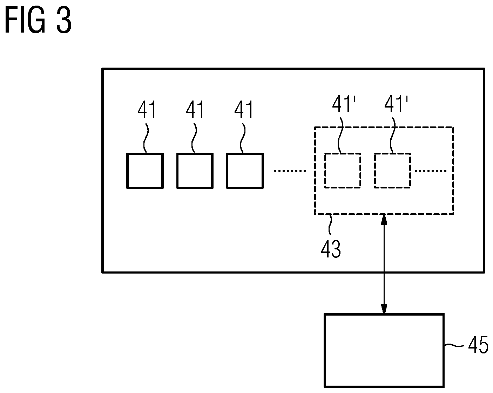

Fig. 3 zeigt einen schematischen Überblick über Steuerungsvorgänge, die zur Steuerung einer medizinischen Diagnostik- und/oder Therapieanlage durchgeführt werden.

Bei der Steuerung können verschiedene Steuervorgänge 41, 41' ausgeführt werden. Bei einem Teil 43 der Steuervorgänge können die zu diesem Teil 43 gehörigen Steuervorgänge 41' erst dann ausgeführt werden, wenn eine bestimmte Bedingung 45 vorliegt. Diese Bedingung ist dann gegeben, wenn sich ein mobiles Handbedienteil, mit dem ein steuerbares Element der medizinischen Diagnostik- und/oder Therapieanlage gesteuert wird, an einem vordefinierten Ort innerhalb der medizinischen Diagnostik- und/oder Therapieanlage befindet. Solange diese Bedingung 45 nicht erfüllt ist, sind die zu dem Teil 43 gehörigen Steuervorgänge 41' nicht aktivierbar. Die zu dem Teil 43 gehörigen Steuervorgänge 41' können dabei Steuervorgänge sein, die das steuerbare Element steuern, das ebenso durch das mobile Handbedienteil gesteuert werden kann. Die zu dem Teil gehörigen Steuervorgänge können dabei auch Steuervorgänge sein, mit denen weitere steuerbare Elemente gesteuert werden, deren Steuerung nicht durch das mobile Handbedienteil vorgenommen werden kann.

## Patentansprüche

1. Steuerungsvorrichtung für eine Partikeltherapieanlage (10), umfassend
- eine Steuerungseinheit (23), mit welcher Steuervorgänge (41, 41') zur Steuerung der Partikeltherapieanlage durchführbar sind, wobei die Steuerungseinheit (23) zur Abgabe eines Strahlfreigabesignals ausgebildet ist,
- ein mobiles Handbedienteil (33), mit dem zumindest ein steuerbares Element der Partikeltherapieanlage durch einen Anwender (31) steuerbar ist,
wobei die Steuerungsvorrichtung derart ausgebildet ist,
dass ein Teil (43) der Steuervorgänge (41'), welcher die Abgabe des Strahlfreigabesignals umfasst, erst dann durch die Steuerungseinheit (23) durchführbar ist, wenn sich das mobile Handbedienteil (33) an einem vordefinierten Ort befindet, wobei der vordefinierte Ort einem Platz in einer Halterungsvorrichtung (35) für das mobile Handbedienteil (33) entspricht.

2. Steuerungsvorrichtung nach Anspruch 1, wobei die Steuerungsvorrichtung ein Positionsüberwachungssystem (37) umfasst, mit welchem ermittelbar ist, ob sich das mobile Handbedienteil (33) an dem vordefinierten Ort befindet.

3. Steuerungsvorrichtung nach Anspruch 1 oder 2, wobei das mobile Handbedienteil (33) als schnurloses Handbedienteil ausgebildet ist.

4. Steuerungsvorrichtung nach einem der Ansprüche 1 bis 3, wobei
der Teil (43) der Steuervorgänge (41'), der durch die Steuerungseinheit (23) erst dann durchführbar ist, wenn sich das mobile Handbedienteil (33) an dem vordefinierten Ort befindet, eine Steuerung des steuerbaren Elements (27) umfasst, das mit dem mobilen Handbedienteil (33) durch einen Anwender (31) steuerbar ist.

5. Steuerungsvorrichtung nach einem der Ansprüche 1 bis 4, wobei
der Teil (43) der Steuervorgänge (41'), der durch die Steuerungseinheit (23) erst dann durchführbar ist, wenn sich das mobile Handbedienteil (33) an dem vordefinierten Ort befindet, eine Steuerung von einem weiteren steuerbaren Element umfasst, das nicht durch das mobile Handbedienteil (33) steuerbar ist.

6. Partikeltherapieanlage (10), umfassend
eine Steuerungsvorrichtung nach einem der Ansprüche 1 bis 5 sowie eine Diagnostik- und/oder Therapievorrichtung, die von der Steuerungsvorrichtung steuerbar ist und die das steuerbare Element umfasst.

7. Verfahren zur Steuerung einer Partikeltherapieanlage (10), bei der Steuervorgänge (41, 41') zur Steuerung der Partikeltherapieanlage durchgeführt werden, wobei einer der Steuervorgänge eine Strahlfreigabe ist, wobei ein Teil (43) der Steuervorgänge (41'), welcher die Strahlfreigabe umfasst, erst dann durchführbar ist, wenn sich ein mobiles Handbedienteil (33), mit dem zumindest ein steuerbares Element der Partikeltherapieanlage durch einen Anwender (31) steuerbar ist, an einem vordefinierten Ort befindet, wobei der vordefinierte Ort einem Platz in einer Halterungsvorrichtung (35) für das mobile Handbedienteil (33) entspricht.

8. Verfahren nach Anspruch 7, wobei
die Position des mobilen Handbedienteils (33) überwacht wird und festgestellt wird, ob sich das mobile Handbedienteil (33) an dem vordefinierten Ort befindet.

9. Verfahren nach Anspruch 7 oder 8, wobei
das mobile Handbedienteil (33) als schnurloses Handbedienteil ausgebildet ist.

10. Verfahren nach einem der Ansprüche 7 bis 9, wobei der Teil (43) der Steuervorgänge (41'), der erst dann durchführbar ist, wenn sich das mobile Handbedienteil (33) an einem vordefinierten Ort befindet, eine Steuerung des steuerbaren Elements umfasst, das mit dem mobilen Handbedienteil (33) durch einen Anwender (31) steuerbar ist.

11. Verfahren nach einem der Ansprüche 7 bis 10, wobei der Teil (43) der Steuervorgänge (41), der durch die Steuerungseinheit erst dann durchführbar ist, wenn sich das mobile Handbedienteil (33) an dem vordefinierten Ort befindet, eine Steuerung von einem weiteren steuerbaren Element umfasst, das nicht durch das mobile Handbedienteil (33) steuerbar ist.

## Claims

1. A control apparatus for a particle therapy system (10) comprising
- a control unit (23) by which control processes (41, 41') for controlling the particle therapy system are performable, wherein the control unit (23) is configured to output a beam release signal;
- a mobile manual control unit (33) by which at least one controllable element of the particle therapy system is controllable by a user (31),
wherein the control apparatus is configured such that a portion (43) of the control processes (41') that comprises the output of the beam release signal is only performable by the control unit (23) when the mobile manual control unit (33) is located at a predefined location, with the predefined location corresponding to a position in a holder apparatus (35) for the mobile manual control unit (33).

2. A control apparatus in accordance with claim 1, wherein the control apparatus comprises a position monitoring system (37) by which it can be determined whether the mobile manual control unit (33) is located at the predefined location.

3. A control apparatus in accordance with claim 1 or claim 2, wherein the mobile manual control unit (33) is configured as a cordless manual control unit.

4. A control apparatus in accordance with one of the claims 1 to 3, wherein the portion (43) of the control processes (41') that is only performable by the control unit (23) when the mobile manual control unit (33) is located at the predefined location comprises a control of the controllable element (27) that is controllable by a user (31) using the mobile manual control unit (33).

5. A control apparatus in accordance with one of the claims 1 to 4, wherein the portion (43) of the control processes (41') that is only performable by the control unit (23) when the mobile manual control unit (33) is located at the predefined location comprises a control of a further controllable element that is not controllable by the mobile manual control unit (33).

6. A particle therapy system (10) comprising a control apparatus in accordance with one of the claims 1 to 5 and a diagnostic and/or therapeutic apparatus that is controllable by the control apparatus and that comprises the controllable element.

7. A method of controlling a particle therapy system (10) in which control processes (41, 41') for controlling the particle therapy system are performed, wherein one of the control processes is a beam release, with a portion (43) of the control processes (41') that comprises the beam release only being performable when a mobile manual control unit (33) by which at least one controllable element of the particle therapy system is controllable by a user (31) is located at a predefined location, with the predefined location corresponding to a position in a holder apparatus (35) for the mobile manual control unit (33).

8. A method in accordance with claim 7, wherein the position of the mobile manual control unit (33) is monitored and a determination is made whether the mobile manual control unit (33) is located at the predefined location.

9. A method in accordance with claim 7 or claim 8, wherein the mobile manual control unit (33) is configured as a cordless manual control unit.

10. A method in accordance with one of the claims 7 to 9, wherein the portion (43) of the control processes (41') that is only performable when the mobile manual control unit (33) is located at a predefined location comprises a control of the controllable element (31) that is controllable by a user (31) using the mobile manual control unit (33).

11. A method in accordance with one of the claims 7 to 10, wherein the portion (43) of the control processes (41') that is only performable by the control unit when the mobile manual control unit (33) is located at the predefined location comprises a control of a further controllable element that is not controllable by the mobile manual control unit (33).

## Revendications

1. Dispositif de commande destiné à une installation d'hadronthérapie (10), comprenant
- une unité de commande (23), au moyen de laquelle des processus de commande (41, 41') peuvent être exécutés pour la commande de l'installation d'hadronthérapie, l'unité de commande (23) étant conçue pour émettre un signal de déblocage de rayon,
- un organe mobile de commande manuelle (33), au moyen duquel au moins un élément pouvant être commandé de l'installation d'hadronthérapie peut être commandé par un utilisateur (31),
le dispositif de commande étant conçu de telle manière qu'une partie (43) des processus de commande (41'), qui comprend l'émission du signal de déblocage de rayon, peut être exécutée par l'unité de commande (23) uniquement quand l'organe mobile de commande manuelle (33) se trouve dans un lieu prédéfini, le lieu prédéfini correspondant à un emplacement dans un dispositif de support (35) pour l'organe mobile de commande manuelle (33).

2. Dispositif de commande selon la revendication 1, comprenant un système de surveillance de position (37), au moyen duquel il est possible de déterminer si l'organe mobile de commande manuelle (33) se trouve dans le lieu prédéfini.

3. Dispositif de commande selon la revendication 1 ou 2, dans lequel l'organe mobile de commande manuelle (33) est réalisé sous la forme d'un organe de commande manuelle sans fil.

4. Dispositif de commande selon l'une des revendications 1 à 3, dans lequel la partie (43) des processus de commande (41'), qui peut être exécutée par l'unité de commande (23) uniquement quand l'organe mobile de commande manuelle (33) se trouve dans le lieu prédéfini, comprend une commande de l'élément (27) pouvant être commandé, qui peut être commandé par un utilisateur (31) au moyen de l'organe mobile de commande manuelle (33).

5. Dispositif de commande selon l'une des revendications 1 à 4, dans lequel la partie (43) des processus de commande (41'), qui peut être exécutée par l'unité de commande (23) uniquement quand l'organe mobile de commande manuelle (33) se trouve dans le lieu prédéfini, comprend une commande d'un autre élément pouvant être commandé, qui ne peut pas être commandé par l'organe mobile de commande manuelle (33).

6. Installation d'hadronthérapie (10), comprenant un dispositif de commande selon l'une des revendications 1 à 5, ainsi qu'un dispositif de diagnostic et/ou de thérapie, qui peut être commandé par le dispositif de commande et qui comprend l'élément pouvant être commandé.

7. Procédé de commande d'une installation d'hadronthérapie (10), dans lequel des processus de commande (41, 41') sont exécutés pour commander l'installation d'hadronthérapie, un des processus de commande étant un déblocage de rayon, une partie (43) des processus de commande (41'), qui comprend le déblocage de rayon, pouvant être exécutée uniquement quand un organe mobile de commande manuelle (33), au moyen duquel au moins un élément pouvant être commandé de l'installation d'hadronthérapie peut être commandé par un utilisateur (31), se trouve dans un lieu prédéfini, le lieu prédéfini correspondant à un emplacement dans un dispositif de support (35) pour l'organe mobile de commande manuelle (33).

8. Procédé selon la revendication 7, dans lequel la position de l'organe mobile de commande manuelle (33) est surveillée et il est déterminé si l'organe mobile de commande manuelle (33) se trouve dans le lieu prédéfini.

9. Procédé selon la revendication 7 ou 8, dans lequel l'organe mobile de commande manuelle (33) est réalisé sous la forme d'un organe de commande manuelle sans fil.

10. Procédé selon l'une des revendications 7 à 9, dans lequel la partie (43) des processus de commande (41'), qui peut être exécutée uniquement quand l'organe mobile de commande manuelle (33) se trouve dans un lieu prédéfini, comprend une commande de l'élément pouvant être commandé, qui peut être commandé par un utilisateur (31) au moyen de l'organe mobile de commande manuelle (33).

11. Procédé selon l'une des revendications 7 à 10, dans lequel la partie (43) des processus de commande (41), qui peut être exécutée par l'unité de commande uniquement quand l'organe mobile de commande manuelle (33) se trouve dans le lieu prédéfini, comprend une commande d'un autre élément pouvant être commandé, qui ne peut pas être commandé par l'organe mobile de commande manuelle (33).
